# EUROPEAN PATENT APPLICATION

(11) **EP 2 264 034 A1**
(43) Date of publication of application: **22.12.2010**
(21) Application number: 09721067.8
(22) Date of filing: 03.03.2009
(51) Int. Cl.: C07D 487/04, A61K 31/519, A61P 1/04, A61P 3/10, A61P 13/06, A61P 19/02, A61P 19/10, A61P 29/00, A61P 31/18, A61P 35/00, A61P 37/02, A61P 37/06, A61P 43/00

(54) **BICYCLIC PYRROLE COMPOUND**

(30) Priority: 10.03.2008 JP 2008059471
(71) Applicant: Dainippon Sumitomo Pharma Co., Ltd., Osaka 541-8524 (JP)
(72) Inventor: SAKURAI, Yasumitsu, Osaka-shi Osaka 554-0022 (JP); NISHIO, Yukihiro, Osaka-shi Osaka 554-0022 (JP); NAKAHIRA, Hiroyuki, Osaka-shi Osaka 554-0022 (JP)
(74) Representative: Duckworth, Timothy John
(86) International application number: PCT/JP2009/053910
(87) International publication number: WO 2009/113423

(57) **Abstract**

Disclosed is a compound composed of an easily-handleable crystal form and having storage stability that is practical enough to be used as an active ingredient of a pharmaceutical product. Specifically disclosed is 6-[(3R)-3-aminopiperidin-1-yl]-5-(2-chloro-5-fluorobenzyl)-1,3-dimethyl-1H-pyrrolo[3,2-d]pyrimidine-2,4(3H,4H)-dione monohydrochloride hemihydrate.

## Description

### TECHNICAL FIELD

The present invention relates to 6-[(3R)-3-aminopiperidin-1-yl]-5-(2-chloro-5-fluorobenzyl)-1,3-dimethyl-1H-pyrrolo[3,2-d]pyrimidine-2,4(3H,5H)-dione monohydrochloride hemihydrate (hereinafter sometimes abbreviated as a compound A where necessary). The compound of the present invention is useful as a pharmaceutical product which can be put into practical use as pharmaceutical bulk drugs, and more particularly, is effective as a dipeptidyl peptidase-IV (DPP-IV) inhibitor. The present invention further relates to a therapeutic agent for diabetes containing a compound of the present invention as an active ingredient.

### BACKGROUND ART

In PATENT DOCUMENT 1, a compound of Example 6 represented by formula (I) (hereinafter referred to as a compound B is disclosed.

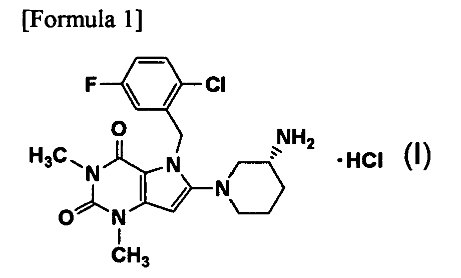

The compound B has an excellent inhibitory action on dipeptidyl peptidase-IV (DPP-IV) and the utility as a therapeutic agent for diabetes can be expected. However, in order to put into practice the present compound as a drug substance of a pharmaceutical product, improvement is required with respect to storage stability and handling.
PATENT DOCUMENT 1: International Publication No. 2006/068163 pamphlet

### DISCLOSURE OF THE INVENTION

### PROBLEMS TO BE SOLVED BY THE INVENTION

The object of the present invention is to provide a compound, of which storage stability is maintained so as to be put into practical use as pharmaceutical bulk drugs and which is composed of a crystalline form that can be easily handled.

### MEANS FOR SOLVING THE PROBLEMS

As a result of the earnest studies on the compound B, the present inventors have found that the compound A, which is a 0.5 hydrate of the compound B, is obtained as a crystal showing excellent stability. The present inventors have found that the compound A is significantly improved in storage stability and handling easiness as compared with the compound B and is extremely useful in that it is put into practical use as a pharmaceutical product, and have reached the completion of the present invention.

That is, the present invention relates to:
[1] 6-[(3R)-3-aminopiperidin-1-yl]-5-(2-chloro-5-fluorobenzyl)-1,3-dimethyl-1H-pyrrolo[3,2-d]pyrimidine-2,4(3H,5H)-dione monohydrochloride hemihydrate,
[2] the compound according to [1], which shows main peaks at diffraction angles (20) in powder X-ray diffraction: 8.6±0.2°, 14.9±0.2° and 15.4±0.2°,
[3] the compound according to [1], which shows main peaks at diffraction angles (20) in powder X-ray diffraction: 4.3±0.2°, 8.6±0.2°, 12.9±0.2°, 14.9±0.2°, 15.4t0.2°, 17.1±0.2°, 18.7±0.2°, 23.0±0.2°, 25.2±0.2°, 26.4±0.2°, 30.3±0.2° and 34.7±0.2°,
[4] a medicament containing a compound described in any of [1] to [3] as an active ingredient,
[5] a dipeptidyl peptidase-IV inhibitor containing a compound described in any of [1] to [3] as an active ingredient,
[6] a therapeutic agent for diabetes containing a compound described in any of [1] to [3] as an active ingredient, and
[7] a method of treating diabetes comprising administering an effective amount of a compound described in any of [1] to [3] to a patient requiring treatment.

### ADVANTAGES OF THE INVENTION

The compound of the present invention is excellent in storage stability and handling easiness and is extremely useful in that it is put into practical use as a pharmaceutical product.

### BRIEF DESCRIPTION OF THE DRAWINGS

Fig. 1 is an X-ray diffraction pattern of a crystal of a monohydrochloride 0.5 hydrate of Example 1 (compound A);

Fig. 2 is a characteristic DSC curve of a crystal of a monohydrochloride 0.5 hydrate of Example 1 (compound A);

Fig. 3 is a characteristic TGA curve of a crystal of a monohydrochloride 0.5 hydrate of Example 1 (compound A);

Fig. 4 is the hygroscopicity of a compound of Example 1 (compound A): ○ represents the weight change under moisture adsorption conditions and ▲ represents the weight change under moisture desorption conditions;

Fig. 5 is the hygroscopicity of a compound of Example 6 of International Publication No. 2006/068163 pamphlet: ○ represents the weight change under moisture adsorption conditions and ▲ represents the weight change under moisture desorption conditions; and

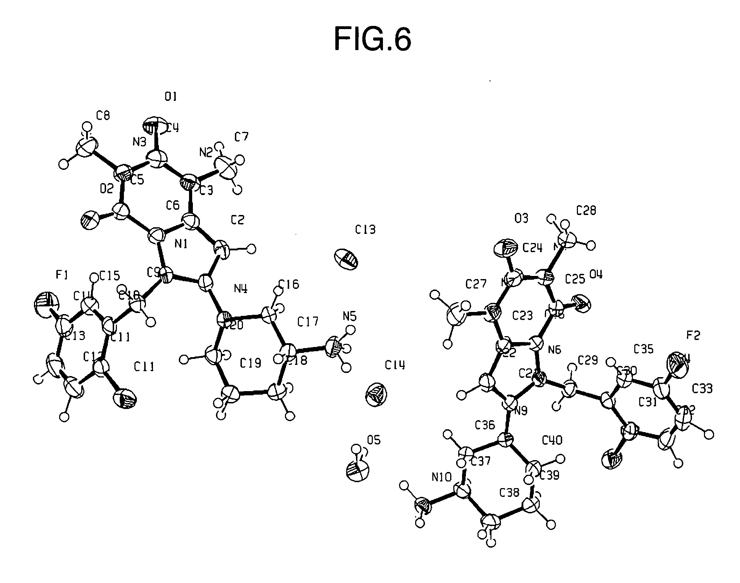

Fig. 6 is the single-crystal X-ray structure analysis of a compound of Example 1 (compound A).

### BEST MODE FOR CARRYING OUT THE INVENTION

The present invention will be described in more detail below.

The compound A is a compound represented by the following formula.

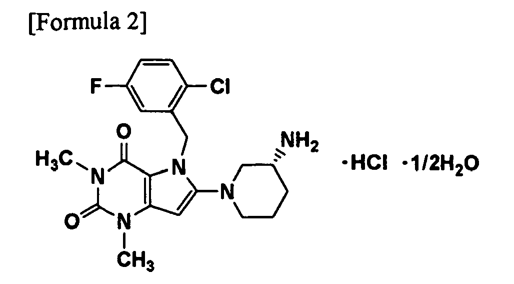

A method of producing the compound of the present invention will be described by citing examples, but the present invention is not intended to be limited by these examples. In addition, in the present description, for the simplification of description, the following abbreviations may be used. Me: a methyl group, Et: an ethyl group, ^{t}Bu: a tert-butyl group, Boc: a tert-butoxycarbonyl group.

The compound of the present invention may be synthesized from a well-known compound by a combination of well-known synthetic methods. For example, the compound may be synthesized according to the methods described in International Publication No. 2006/068163 pamphlet. One of the examples is shown below.

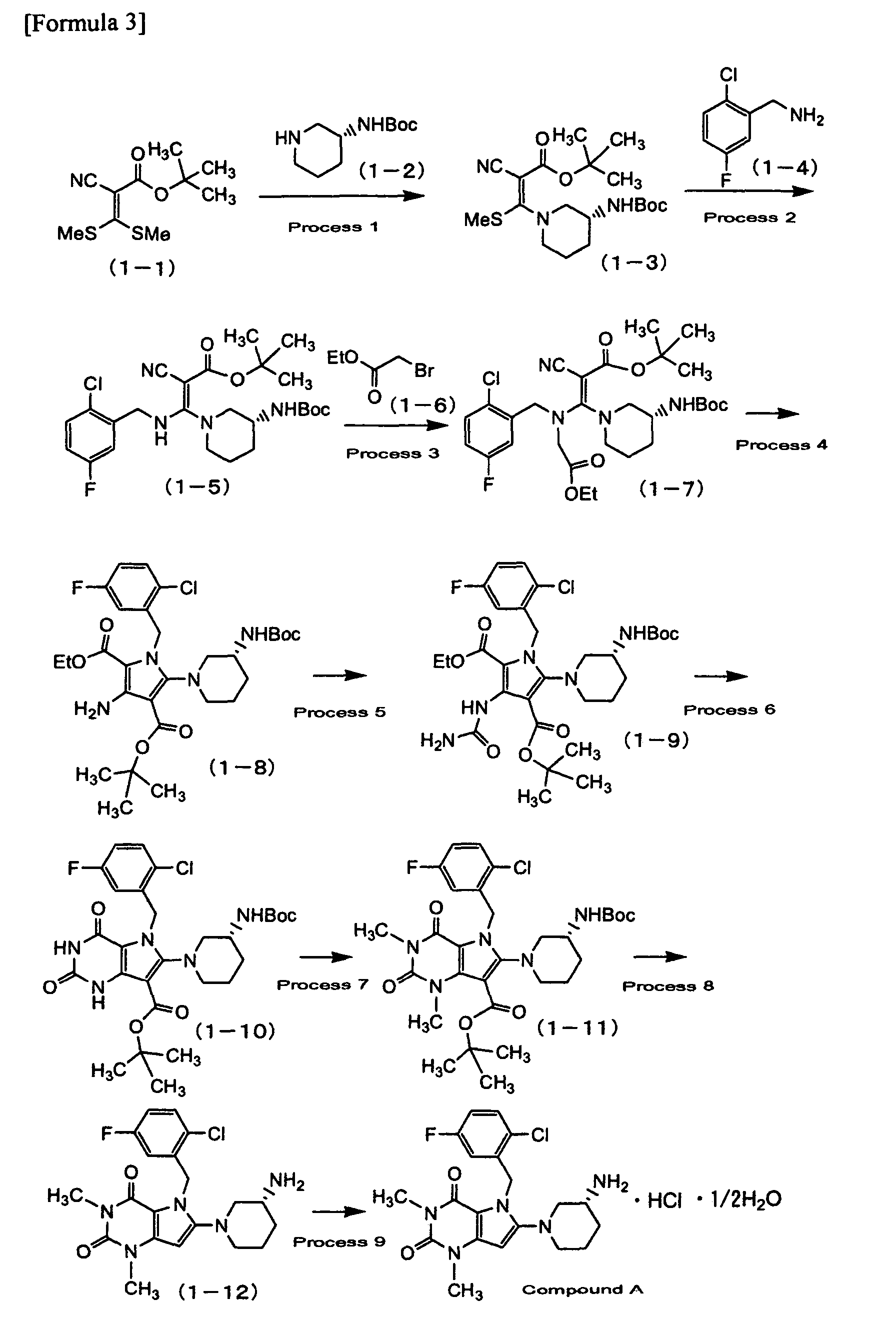

### 1) Process 1

A compound (1-3) can be produced by the reaction of a compound (1-1) with a compound (1-2) in an inert solvent. Examples of the inert solvent include, for example, an aromatic hydrocarbon solvent such as toluene, benzene or xylene. The reaction temperature may be generally selected from the range of approximately 20°C to approximately 100°C.

### 2) Process 2

A compound (1-5) is produced by the reaction of the compound (1-3) with a compound (1-4) in an inert solvent in the presence of an organic base. Examples of the inert solvent include a nitrile solvent such as acetonitrile or propionitrile. Examples of the organic base include 1,8-diazabicyclo[5.4.0]undeca-7-ene (DBU), 1,5-diazabicyclo[4.3.0]nona-5-ene or 1,4-diazabicyclo[5.4.0]undeca-7-ene. The reaction temperature is generally selected from the range of approximately 20°C to approximately 100°C, but the reaction may be carried out under reflux.

### 3) Process 3

A compound (1-7) can be produced by the reaction of the compound (1-5) with a compound (1-6) in an inert solvent in the presence of a base. Examples of the base include, for example, an alkali carbonate such as potassium carbonate, sodium carbonate, cesium carbonate, potassium bicarbonate or sodium bicarbonate, and preferably potassium carbonate. Examples of the inert solvent include a non-protonic solvent (such as N,N-dimethylformamide or dimethyl sulfoxide), an ether solvent (such as diethyl ether, tetrahydrofuran or 1,4-dioxane), a ketone (such as acetone) or a mixed solvent thereof, and preferably N,N-dimethylformamide. The reaction temperature is generally selected from the range of approximately 10°C to approximately 80°C.

### 4) Process 4

A compound (1-8) can be produced by the reaction of the compound (1-7) with a base in an inert solvent. Examples of the base include an alkali amide such as lithium amide or sodium amide or an alkali hydride such as sodium hydride or potassium hydride. Examples of the inert solvent include an aromatic hydrocarbon solvent such as toluene, benzene or xylene, a hydrocarbon solvent such as hexane or heptane, tert-butylalcohol, acetonitrile, N,N-dimethylformamide, an ether solvent (such as diethyl ether, tetrahydrofuran or 1,4-dioxane) or a mixed solvent thereof. As the base, preferred is lithium amide. Examples of the solvent preferably include a mixed solvent of tert-butylalcohol, acetonitrile and heptane or a mixed solvent of tert-butylalcohol, acetonitrile, heptane and toluene. The reaction temperature may be generally selected from the range of approximately 10°C to approximately 120°C.

### 5) Process 5

A compound (1-9) is produced by the reaction of the compound (1-8) with potassium cyanate in an inert solvent. Examples of the inert solvent include water, an organic acid such as acetic acid or propionic acid, or an aromatic hydrocarbon solvent such as toluene, benzene or xylene, and the reaction is generally carried out in a mixed solvent of water, acetic acid and toluene. The reaction temperature is generally selected from the range of approximately 20°C to approximately 80°C.

### 6) Process 6

A compound (1-10) is produced by the reaction of the compound (1-9) with an inorganic base in an inert solvent. Examples of the inorganic base include potassium carbonate, cesium carbonate or sodium carbonate. Examples of the inert solvent include water or a non-protonic solvent (such as N,N-dimethylformamide or dimethylsulfoxide), and the reaction is generally carried out in a mixed solvent of water and N,N-dimethylformamide. The reaction temperature is generally selected from the range of approximately 20°C to approximately 100°C.

### 7) Process 7

A compound (1-11) is produced by the reaction of the compound (1-10) with methyl iodide in an inert solvent in the presence of an inorganic base. Examples of the inorganic base include potassium carbonate, cesium carbonate or sodium carbonate. Examples of the inert solvent include a non-protonic solvent (such as N,N-dimethylformamide or dimethylsulfoxide) and N,N-dimethylformamide is preferably used. The reaction temperature is generally selected from the range of approximately 20°C to approximately 50°C. In addition, the reaction can be carried out by adding methyl iodide into the reaction solution in the process 6.

### 8) Process 8

A compound (1-12) is produced by the reaction of the compound (1-11) with an inorganic acid in an inert solvent. Examples of the inorganic acid include hydrochloric acid or sulfuric acid and sulfuric acid is preferably used. Examples of the inert solvent include an ether solvent (such as diethyl ether, 1,2-dimethoxyethane, diglyme, tetrahydrofuran or 1,4-dioxane) or a mixed solvent thereof, and tetrahydrofuran is preferably used. The reaction temperature is generally selected from the range of approximately 30°C to approximately 120°C. After completion of the reaction, an organic solvent 1 and water are added to the reaction solution, followed by liquid separation. The inorganic base, water and an organic solvent 2 are added into the water layer, followed by liquid separation (operation 1). The organic solvent 2 is added again into the water layer, followed by liquid separation (operation 2). An organic layer obtained in operations 1 and 2 is concentrated under reduced pressure. An alcohol is added into the resulting residue, followed by heating within the range of approximately 50°C to approximately 100°C. Water is added dropwise into the resulting mixture. After completion of the dropwise addition, the mixture is cooled within the range of approximately 10°C to approximately 40°C, followed by stirring within the range of approximately -5°C to approximately 10°C. The resulting solid is obtained by filtration and washed with water, followed by drying under reduced pressure. Examples of the inorganic base include sodium hydroxide or potassium hydroxide. Examples of the organic solvent 1 include an aromatic hydrocarbon solvent such as toluene, benzene or xylene and toluene is preferably used. Examples of the organic solvent 2 include an ether solvent such as tetrahydrofuran or methyl tert-butyl ether and tetrahydrofuran is preferably used. Examples of the alcohol include propanol, butanol, pentanol, hexanol, heptanol or octanol and isopropanol is preferably used.

### 9) Process 9

The compound A is produced by adding hydrochloric acid into an aqueous solution of the compound (1-12). After completion of the reaction, toluene is added into the reaction solution, followed by concentration under reduced pressure. The operation of the concentration under reduced pressure may be carried out a plurality of times. Hydrous alcohol is added into the resulting residue, followed by heating within the range of approximately 50°C to approximately 120°C. Ethyl acetate is added dropwise into the resulting mixture. After completion of the dropwise addition, the mixture is cooled within the range of approximately 10°C to approximately 40°C, followed by stirring within the range of approximately 0°C to approximately 20°C. The resulting solid is obtained by filtration and washed with ethyl acetate, followed by drying under reduced pressure. Examples of the hydrous alcohol include propanol, butanol, pentanol, hexanol, heptanol or octanol and isopropanol is preferably used. The proportion of water in the hydrous alcohol is in the range of approximately 5% to approximately 30% and preferably approximately 10% to approximately 20% in a volume ratio to the alcohol.

The raw materials or reagents used in the above processes are commercially available compounds unless otherwise specified and may be produced from well-known compounds using well-known methods. For example, the compound (1-2) can be synthesized according to the method described in PATENT DOCUMENT (JP2007-262040A).

In addition, the compound of the present invention is synthesized as a racemic form and can also be preparatively isolated by column chromatography using a filler to which an optically active ligand is bonded.

The compound of the present invention is applied to the treatment of various diseases due to the inhibitory action on DPP-IV. The compounds described in the present description are useful for the suppression of postprandial hyperglycemia in the prediabetic state, the treatment of non-insulin-dependent diabetes mellitus, the treatment of autoimmune diseases such as joint inflammation or rheumatoid arthritis, the treatment of intestinal mucosal diseases, the promotion of growth, the prevention of rejection of a transplanted organ, the treatment of obesity, the treatment of an eating disorder, the treatment of HIV infection, the suppression of cancer metastasis, the treatment of prostatic hypertrophy, the treatment of periodontitis and the treatment of osteoporosis.

When used for treatment purposes, the compound of the present invention can be administered orally or parenterally (for example, intravenous, subcutaneous or intramuscular injection, locally, rectally, transdermally or nasally) as a pharmaceutical composition. Examples of the composition for oral administration include, for example, tablets, capsules, pills, granules, powders, liquids and suspensions. Examples of the composition for partenteral administration include, for example, aqueous preparations or oil preparations for injection, ointments, creams, lotions, aerosols, suppositories and plasters. These preparations are prepared using conventionally well-known techniques and can contain a nontoxic and inactive carrier or diluent which is generally used in the field of preparations.

The dosage varies depending on each compound and the disease, age, body weight, sex, symptom, administration route and the like of patients. The compound of the present invention is administered generally at a dose of 0.1 to 1000 mg/day and preferably 1 to 300 mg/day once a day or in two to three divided doses, based on an adult (body weight of 50 kg). In addition, the compound may be administered once in a few days to few weeks.

For the purpose of increasing the effect of the compound of the present invention, the compound can be used in a combination with a pharmaceutical preparation (hereinafter abbreviated as a combined drug) such as a therapeutic agent for diabetes, a therapeutic agent for diabetic complications, an antilipidemic agent, an antihypertensive agent, an antiobesis agent and a diuretic agent. The administration time of the compound of the present invention and the combined drug is not limited, and the compound and the preparation may be administered simultaneously or separately at an interval to the subject to be administered. In addition, a combined preparation of the compound of the present invention and the combined drug may be prepared. The dosage of the combined drug can be arbitrarily selected based on the clinically used dosage. Further, the blending ratio of the compound of the present invention to the combined drug can be arbitrarily selected depending on the subject to be administered, administration route, diseases to be covered, symptom and a combination. For example, when a subject to be administered is a human being, 0.01 to 100 parts by weight of the combined preparation may be used based on 1 part by weight of the compound of the present invention.

In addition, examples of the therapeutic agent for diabetes include an insulin preparation (for example, an animal insulin preparation extracted from a bovine or porcine pancreas and a human insulin preparation synthesized by genetic engineering using Escherichia coli and yeast), an insulin resistance improving agent (for example, pioglitazone and a hydrochloride thereof, triglitazone, rosiglitazone or a maleate salt thereof, GI-262570, JTT-501, MCC-555, YM-440, KRP-297 and CS-011), an α-glycosidase inhibitor (for example, voglibose, acarbose, miglitol and emiglitate), a biguanide agent (for example, metoformin), an insulin secretion promoter (for example, a sulfonylurea agent such as tolbutamide, glibenclamide, gliclazide, chlorpropamide, trazamide, acetohexamide, glyclopyramide and glimepiride; repaglinide, senaglinide, nateglinide and mitiglinide), GLP-1, a GLP-1 analog (such as exenatide, liraglutide, SIJN-E7001, AVE010, BIM-51077 and CJC1131), a protein tyrosine phosphatase inhibitor (for example, vanadic acid) and a β3 agonist (for example, GW-427353B and N-5984).

Examples of the therapeutic agent for diabetic complications include an aldose reductase inhibitor (for example, tolrestat, epalrestat, zenarestat, zopolrestat, minarestat, fidarestat, ranirestat, SK-860 and CT-112), a neurotrophic factor (for example, NGF, NT-3 and BDNF), a PKC inhibitor (for example, LY-333531), an AGE inhibitor (for example, ALT946, pimagedine, pyratoxatin, N-phenacylthiazolium bromide (ALT-766)), an active oxygen scavenger (for example, thioctic acid) and a cerebral vasodilator (for example, tiapride and mexiletine). Examples of the antilipidemic agent include an HMG-CoA reductase inhibitor (for example, pravastatin, simvastatin, lovastatin, atorvastatin, fluvastatin, itavastatin or a sodium salt thereof), a squalene synthetase inhibitor and an ACAT inhibitor. Examples of antihypertensive agent include an angiotensin convertase inhibitor (for example, captopril, enalapril, alacepril, delapril, lisinopril, imidapril, benazepril, cilazapril, temocapril and trandolapril), an angiotensin II antagonist (for example, olmesartan, medoxomil, candesartan, cilexetil, losartan, eprosartan, valsartan, telmisartan, irbesartan and tasosartan), a calcium antagonist (for example, nicardipine hydrochloride, manidipine hydrochloride, nisoldipine, nitrendipine, nilvadipine and amlodipine) and a renin inhibitor (such as aliskiren).

Examples of the antiobesity agent include, for example, a central antiobesity agent (for example, phentermine, sibutramine, amfepramone, dexamfetamine, mazindol, SR-141716A), a pancreatic lipase inhibitor (for example, orlistat), a peptidic anorectic drug (for example, leptin, CNTF (ciliary neurotrophic factor)) and a cholecystokinin agonist (for example, lintitript, FPL-15849). Examples of the diuretic agent include, for example, a xanthine derivative (for example, theobromine sodium salicylate and theobromine calcium salicylate), a thiazide preparation (for example, ethiazide, cyclopenthiazide, trichloromethiazide, hydrochlorothiazide, hydroflumethiazide, bentylhydrochlorothiazide, penflutizide, polythiazide and methyclothiazide), an antialdosterone preparation (for example, spironolactone and triamterene), a carbonic anhydrase inhibitor (for example, acetazolamide), a chlorobenzenesulfonamide preparation (for example, chlorthalidone, mefruside and indapamide), azosemide, isosorbide, etacrynic acid, piretanide, bumetanide and furosemide.

Examples of the combined drugs preferably include GLP-1, a GLP-1 analog, an α-glucosidase inhibitor, a biguanide preparation, an insulin secretagogue and an insulin resistance improving agent. The combined drugs may be used by combining two or more kinds at an optional ratio.

When the compound of the present invention is used in combination with the combined drug, the amounts used of these drugs can be reduced within safe ranges in consideration of the side effects of the drugs. Especially, the dosage of the biguanide preparation can be reduced than the usual dosage. Therefore, the side effects that would be caused by the pharmaceutical preparation can be safely prevented. In addition to that, the dosages of a diabetic complication agent, an antilipidemic agent, an antihypertensive agent and the like may be reduced. As a result, the side effects that would be caused by these pharmaceutical preparations may be effectively prevented.

### EXAMPLES

Hereinafter, the present invention will be described more specifically with reference to Reference Examples, Example and Test Examples, but the present invention is not intended to be limited by these Examples. In addition, the compound names shown in the following Reference Examples and Example do not always conform to IUPAC nomenclature. Further, for the simplification of description, the following abbreviations may be used and the meanings of these abbreviations are the same as described above.

### Example 1

### Reference Example 1

### tert-butyl 3-{(3R)-3-[(tert-butoxycarbonyl)amino]- piperidin-1-yl}-2-cyano-3-(methylthio)acrylate

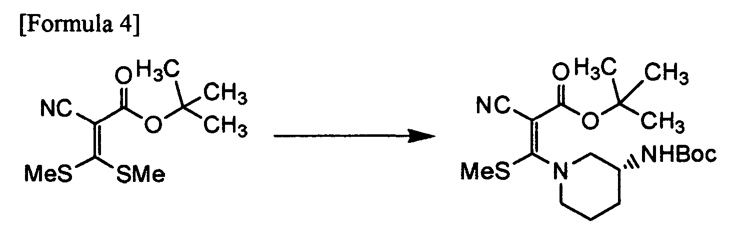

A mixture was prepared by adding a toluene solution (7.40 L) of (3R)-3-[(tert-butoxycarbonyl)amino]-3-methylpiperidine (3.00 kg) into a toluene solution (7.40 L) of tert-butyl 2-cyano-3,3-bis(methylthio)acrylate (3.63 kg) and stirred at 30°C. After 5 hours, hepatane (38.47 L) was added to the mixture and heated at 50°C, followed by cooling to room temperature. Subsequently, the mixture was stirred for one hour in an ice bath and then a precipitated white solid was recovered by filtration, followed by drying under reduced pressure to obtain the title compound (5.00 kg) as a white solid.

MS (ESI+) 398 (M⁺+1, 80%)

### Reference Example 2

### tert-butyl 3-[(2-chloro-5-fluorobenzyl)amino]-3-{(3R)-3-[(tert-butoxycarbonyl)amino]pineridin-1-yl}-2-cyanoacrylate

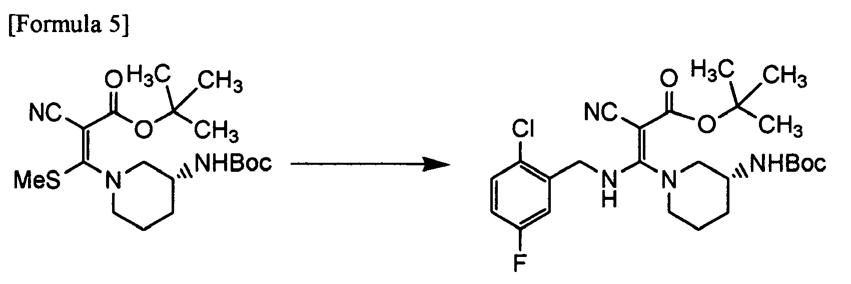

A mixture was prepared by dissolving the compound (5 kg) of Reference Example 1 in acetonitrile (7.50 L), and DBU (3.83 kg) and 2-chloro-5-fluorobenzylamine (2.41 kg) were added, followed by stirring at 50°C. After 6 hours, the mixture was diluted with toluene (14.72 L) and then washed with water. The resulting organic layer was washed with a 10% aqueous solution of potassium bisulfate, a 10% aqueous solution of sodium hydroxide and water, followed by concentration under reduced pressure to obtain the title compound (9.00 kg) as a yellow amorphous substance.

MS (ESI+) 509 (M⁺+1, 50%)

### Reference Example 3

### tert-butyl 3-[(2-chloro-5-fluorobenzyl)(2-ethoxy-2-oxoethyl)amino]-3-{(3R)-3-[(tert-butoxycarbonyl)amino]-piperidin-1-yl}-2-cyanoacrylate

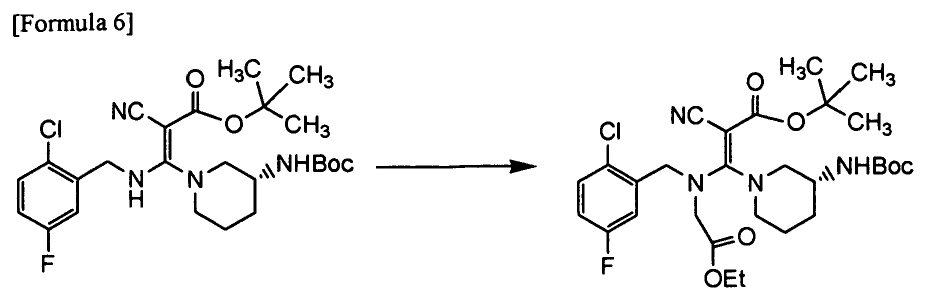

A mixture was prepared by dissolving the compound (98.9 g) of Reference Example 2 in N,N-dimethylformamide (145 mL) and potassium carbonate (96.67 g) was added, followed by stirring at room temperature. To the mixture was added dropwise ethyl bromoacetate (46.72 g), followed by stirring for one hour at 40°C. After adding toluene and filtering through Celite^{®}, the resulting organic layer was washed with water and dried with sodium sulfate and filtered, followed by concentration under reduced pressure to obtain the title compound (135.81 g) as a dark red amorphous substance.

MS (ESI+) 595 (M⁺+1, 40%)

### Reference Example 4

### 4-tert-butyl 2-ethyl 3-amino-1-(2-chloro-5-fluorobenzyl)-5-{(3R)-3-[(tert-butoxycarbonyl)amino]-piperidin-1-yl}-1H-pyrrole-2,4-dicarboxylate

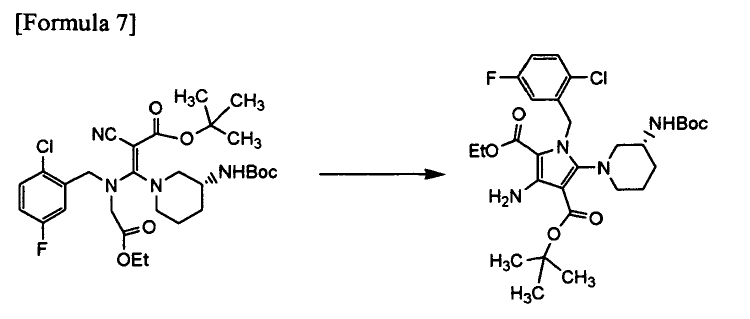

A mixture was prepared by adding heptane (5.75 L) to lithium amide (0.50 kg), and a mixed solution of tert-butylalcohol (12.18 kg) and heptane (1.44 L) was added dropwise at 80°C. After one hour, the mixture was cooled to 30°C and acetonitrile (18.71 L) was added. To the mixture was added a toluene (16.94 L) solution of the compound (10.94 kg) of Reference Example 3, followed by stirring. After 2 hours, the solvents were distilled off under reduced pressure, followed by adding toluene (16.94 L) and water. The pH of the mixture was adjusted to approximately 5.0 with concentrated hydrochloric acid and the mixture was filtered through Celite®, followed by liquid separation. The resulting organic layer was concentrated under reduced pressure to obtain the title compound (9.40 kg) as a dark red oily substance.

MS (ESI+) 595 (M⁺+1, 50%)

### Reference Example 5

### 4-tert-butyl 2-ethyl 3-[(aminocarbonyl)amino]-1-(2-chloro-5-fluorobenzyl)-5-{(3R)-3-[(tert-butoxycarbonyl)amino]-piperidin-1-yl}-1H-pyrrole-2,4-dicarboxylate

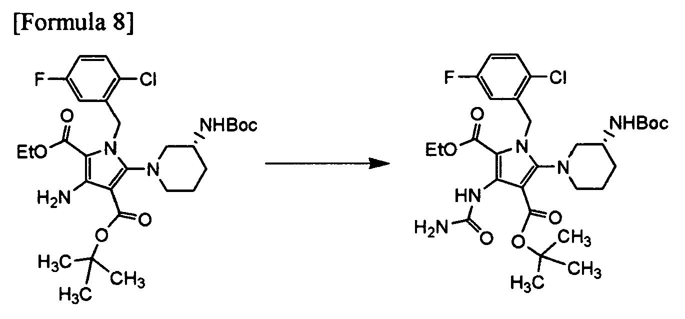

A solution obtained by dissolving potassium cyanate (1.06 kg) into water (2.00 kg) was added dropwise at 40°C into a mixture obtained by dissolving the compound (9.40 kg) of Reference Example 4 into acetic acid (27.80 L) and toluene (15.18 L) and the resulting mixture was stirred. After 2 hours, the mixture was cooled to room temperature and diluted with toluene (700 mL), followed by washing with water three times. The pH of the mixture was adjusted to 7 to 8.5 with an aqueous solution of sodium hydroxide and the mixture was washed with saline solution, followed by concentration under reduced pressure to obtain the title compound (14.48 kg) as a dark red amorphous substance.

MS (ESI+) 638 (M⁺+1, 80%)

### Reference Example 6

### tert-butyl 5-(2-chloro-5-fluorobenzyl)-6-{(3R)-3-[(tert-butoxycarbonyl)amino]-piperidin-1-yl}-1,3-dimethyl-2,4-dioxo-2,3,4,5-tetrahydro-1H-pyrrolo[3,2-d]pyrimidine-7-carboxylate

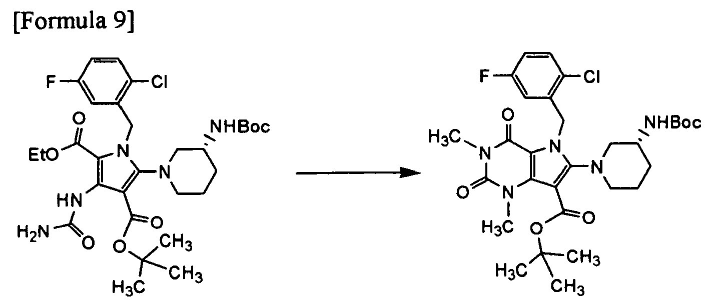

A mixture was prepared by dissolving the compound (14.48 kg) of Reference Example 5 in N,N-dimethylformamide (19.28 L) and potassium carbonate (2.02 kg) and water (0.19 kg) were added, followed by stirring at 50°C. After 5 hours, the mixture was cooled to 30°C and potassium carbonate (2.02 kg) was added. Methyl iodide (2.59 kg) was added dropwise to the mixture and stirred. After 5 hours, the mixture was cooled to room temperature and filtered, and then diluted with toluene, followed by washing with water and concentration under reduced pressure. The resulting solid was heated to 70°C in isopropanol (50.58 L) and cooled to room temperature. After stirring at 5°C for 5 hours, the solid was recovered by filtration, followed by drying under reduced pressure to obtain the title compound (3.835 kg) as a white solid.

MS (ESI+) 620 (M⁺+1, 50%)

### Reference Example 7

### 6-[(3R)-3-aminopiperidin-1-yl]-5-(2-chloro-5-fluorobeoul)-1,3-dimethyl-1H-pyrrolo[3,2-d]pyrimidine-2,4(3H,5H)-dione

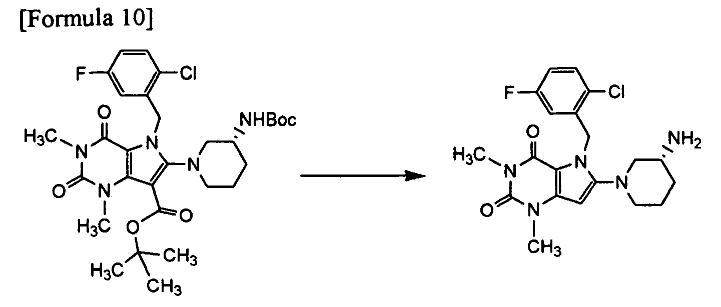

A mixture was prepared by adding dropwise concentrated sulfuric acid (1.26 kg) into tetrahydrofuran (1.50 L). To the mixture was added dropwise a tetrahydrofuran (25.64 L) solution of the compound (3.79 kg) of Reference Example 6 and the mixture was stirred. After 4 hours, the mixture was cooled to room temperature and subjected to liquid separation by adding toluene (25.01 kg) and water (24.07 kg). After the water layer was subjected to liquid separation by adding NaOH (1.17 kg), water (4.69 kg) and tetrahydrofuran (11.37 kg), the water layer was extracted again with tetrahydrofuran. The resulting organic layer was concentrated under reduced pressure and isopropnaol (9.53 kg) was added to the residue, followed by heating to 80°C. To the organic layer was added water (36.79 kg) and stirred for one hour. The organic layer was cooled to room temperature and stirred at 0°C for one hour. A precipitated solid was recovered by filtration and washed with water, followed by drying under reduced pressure to obtain the title compound (2.12 kg) as a white solid.

MS (ESI+) 420 (M+1, 100%)

### Example 1

### 6-[(3R)-3-aminopiperidin-1-yl]-5-(2-chloro-5-fluorobenzyl)-1,3-dimethyl-1H-pyrrolo[3,2-d]pyrimidine-2,4(3H,5H)-dione hydrochloride hemihydrate

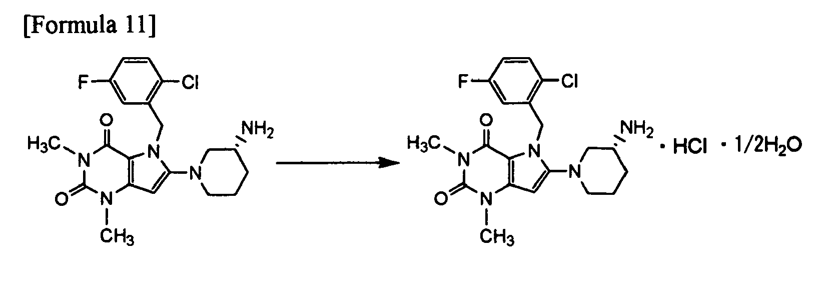

A mixture was prepared by adding 1N hydrochloric acid (2.67 kg) into a mixture of 6-[(3R)-3-aminopiperidin-1-yl]-5-(2-chloro-5-fluorobenzyl)-1,3-dimethyl-1H-pyrrolo[3,2-d]pyrimidine-2,4(3H,5H)-dione (1.02 kg) and water (7.80 kg) and stirred at room temperature. After one hour, to the resulting mixture was added toluene, followed by concentration under reduced pressure three times. To the resulting residue was added isopropanol (4.49 kg) containing 15% of water, followed by heating to 80°C. After a uniform solution was obtained, ethyl acetate (16.64 kg) was added dropwise. After the solution was slowly cooled to room temperature, the solution was stirred for one hour in an ice bath. The resulting solid was recovered by filtration and washed with ethyl acetate, followed by drying under reduced pressure to obtain the title compound (721.8 g) as a white crystal.
¹H NMR (400 MHz, DMSO) δ 8.12 (brs, 3H), 7.53-7.56 (m, 1H), 7.13-7.18 (m, 1H), 6.10-6.15 (m, 2H), 5.39-5.50 (m, 2H), 3.39 (s, 3H), 3.22-3.27 (m, 2H), 3.13 (s, 3H), 2.82-2.88 (m, 2H), 2.66-2.71 (m, 1H), 1.83-1.92 (m, 1H), 1.71-1.78 (m, 1H), 1.40-1.52 (m, 2H).
MS (ESI+) 420 (M⁺+1, 100%).
mp 205-208°C Anal.

| | |
|---|---|
| Found: | C, 51.49; H, 5.39; N, 14.93; Cl, 15.19; F, 3.92 |
| Calculated: | (C₂₀H₂₅Cl₂FN₅O_{2.5}) |
| | C, 51.62; H, 5.41; N, 15.05; Cl, 15.24; F, 4.08 |

The X-ray diffraction pattern analysis of a crystal of a monohydrochloride 0.5 hydrate of Example 1 (compound A) was carried out using X' pert Pro manufactured by Philips Analytical Inc. or an equivalent instrument. CuK_{α1} was used as the X-ray source.

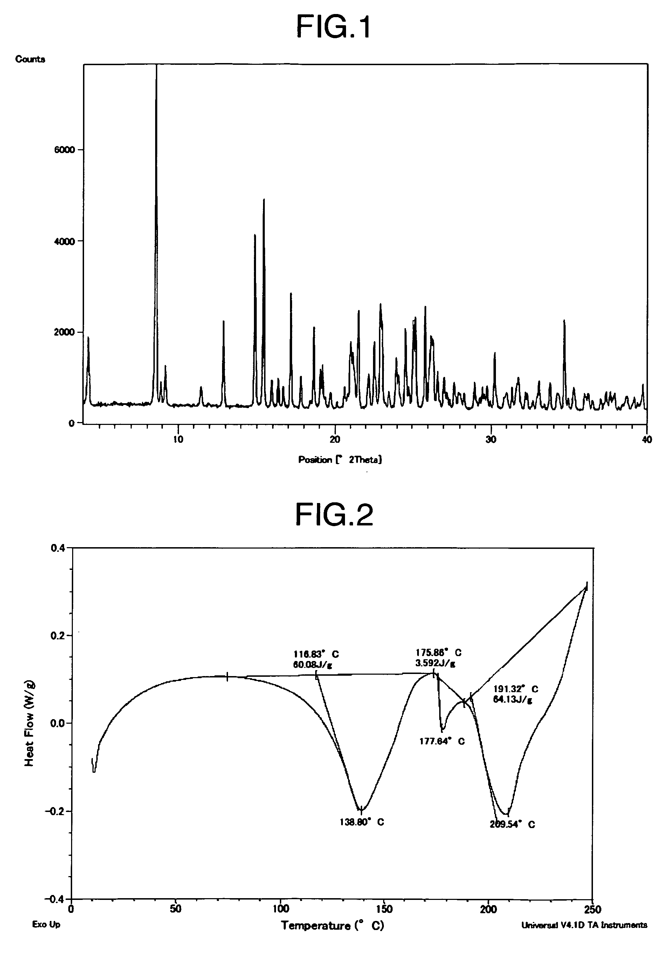

Fig. 1 shows an X-ray diffraction pattern of a crystal of a monohydrochloride 0.5 hydrate of Example 1 (compound A). In the diffraction peak values at diffraction angles 2θ(°) shown below, some measurement errors may occur depending on a measurement instrument or measurement conditions or the like. Specifically, the measurement errors may be within the range of ±0.2 and preferably ±0.1. The 0.5 hydrate (compound A) has characteristic peaks at diffraction angles [2θ(°)] of 4.3°, 8.6°, 12.9°, 14.9°, 15.4°, 17.2°, 18.7°, 23.0°, 25.2°, 26.4°, 30.3° and 34.7°. Among these, further characteristic peaks are 8.6°, 14.9° and 15.4°.

Fig. 2 shows a characteristic DSC curve of a crystal of a monohydrochloride 0.5 hydrate of Example 1 (compound A). DSC Q1000 manufactured by TA Instruments Inc. or an equivalent instrument was used. The measurements were made in the temperature range of 10°C to 250°C using an aluminum hermic pan under a nitrogen flow at 50.0 mL/min. The 0.5 hydrate (compound A) showed characteristic endothermic peaks at around 150°C and 200°C.

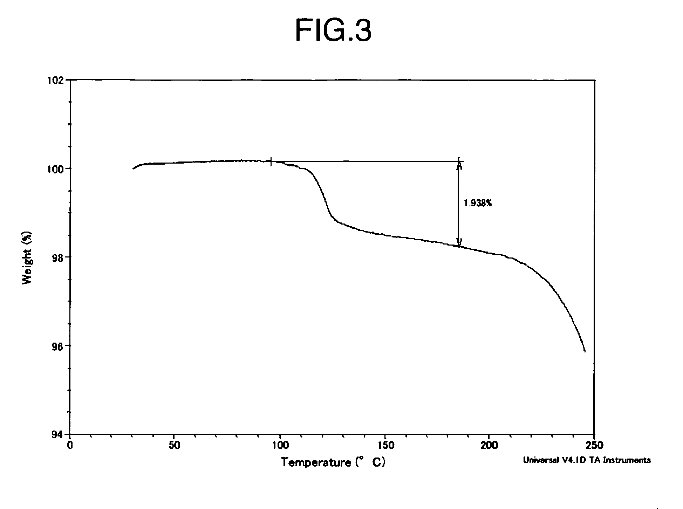

Fig. 3 shows a characteristic TGA curve of a crystal of a monohydrochloride 0.5 hydrate of Example 1 (compound A). TGA Q500 manufactured by TA Instruments Inc. or an equivalent instrument was used. The measurements were made in the temperature range of 10°C to 250°C using a platinum pan under a nitrogen flow at 40.0 mL/min as a balance gas and a nitrogen flow at 60.0 mL/min as a sample gas. The 0.5 hydrate (compound A) showed a weight loss of approximately 2.0% at around 100°C.

### Test Example 1

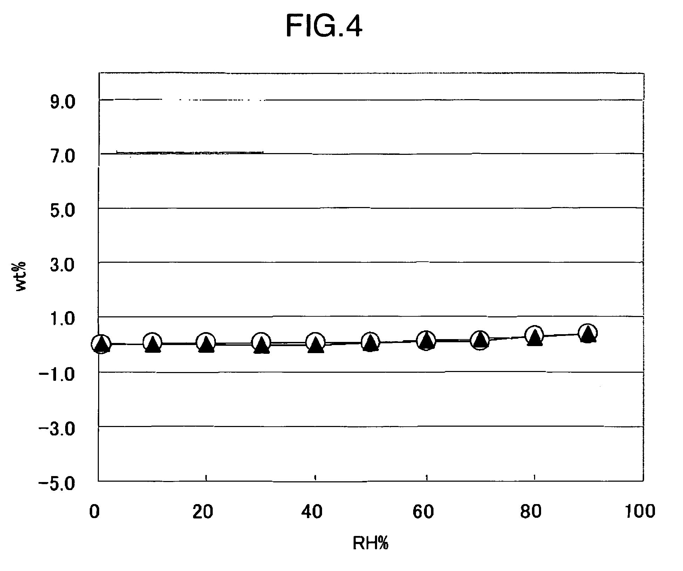

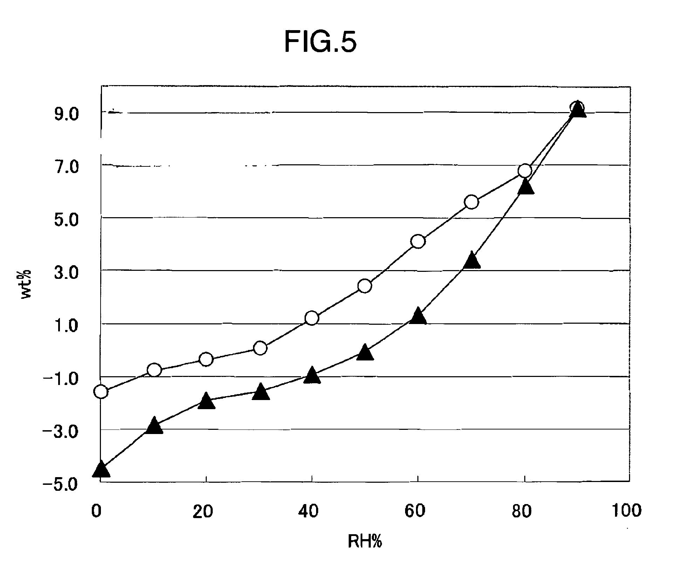

The hygroscopicity was measured at room temperature for each of the crystalline monohydrochloride 0.5 hydrate of Example 1 (compound A) produced by the method of the present invention and the amorphous powders of the compound of Example 6 of International Publication No. 2006/068163 (compound B) produced by a conventional technique. The results are shown in Figs. 4 and 5.

As shown in Fig. 4, in the crystal of the monohydrochloride 0.5 hydrate of Example 1 (compound A), no change in the weight is observed at the humidity of 0 to 90%. On the other hand, as shown in Fig. 5, for the amorphous powders (compound B) produced by a conventional technique, a -5.0 to 9.0% change in the weight was observed at the humidity of 0 to 90%.

### Test Example 2

The residual rates after two week storage under a light source of 1000 lux and 4000 lux were measured for each of the crystalline monohydrochloride 0.5 hydrate of Example 1 (compound A) produced by the method of the present invention and the amorphous powders of the compound of Example 6 of International Publication No. 2006/068163 (compound B) produced by a conventional technique. The results are shown in Table 1.

The residual rate (%) of a compound was determined by the area percentage of the residual rate of the compound measured using high performance liquid chromatography (HPLC). The HPLC measurement conditions were as follows:

Column: CAPCELL PAK C18 UG120 (4.6 mm I.D. × 250 mm)
Mobile phase (A/B): 10 mM sodium phosphate buffer (pH=6.9)/CH₃CN
Mobile phase (ratio of B): 30% (0 min) → 90% (45 min)
Column temperature: 40°C
Flow rate: 1 mL/min
UV wavelength: 254 nm

**[Table 1]**

| | | | |
|---|---|---|---|
| Compound A | Initial | 100.0% | Pale yellow |
| | 1000Lux, 11%RH | 100.0% | Pale yellow |
| | 4000Lux, 10%RH | 99.9% | Pale yellow |
| Compound B | Initial | 100.0% | Pale yellow |
| | 1000Lux, 11%RH | 99.2% | Yellow |
| | 4000Lux, 10%RH | 97.4% | Yellow |

As shown in Table 1, it was observed that the compound of Example 6 in International Publication No. 2006/068163 (compound B) had low stability and was reduced in content under a light source of both 1000 lux and 4000 lux. In contrast, it was confirmed that the compound of Example 1 of the present invention (compound A) had high stability and had extremely high stability even under the conditions of both 1000 lux and 4000 lux. In addition, the compound of Example 1 (compound A) is a pale yellow compound and no change in appearance and no change in color were observed after implementation of the stability test even under the conditions of both 1000 lux and 4000 lux. On the other hand, under the above conditions, the compound of Example 6 in International Publication No. 2006/068163 (compound B) was apparently changed in color to yellow.

### Test Example 3

### Pharmacological Test

### In vitro DPP-IV Inhibitory Action Measurement Test

Solutions containing test compounds were prepared at various concentrations and added to microassay plates. Further, human recombinant DPP IV (R&D system) was added and preincubated at room temperature for 100 to 110 minutes, followed by adding a substrate (Glycyl-L-proline 4-methylcoumaryl-7-amide, Peptide Institute, Inc.; final concentration: 40 µmol/L) to start the enzymatic reaction. The enzymatic reaction rate was determined by measuring the fluorescence change with time for 10 minutes after 10 minutes elapsed from the start of addition of the substrate using a fluorescence plate reader (excitation wavelength: 380 nm, measurement wavelength: 460 nm). From the results obtained, the compound concentration showing 50% inhibition was calculated as an IC₅₀ value.

**[Table 2]**

| Test compound | Human DPP IV inhibitory activity IC₅₀ (nM) |
|---|---|
| Example 1 | 0.71 |

### INDUSTRIAL APPLICABILITY

The present invention can provide a compound which has DPP-IV inhibitory activity and is improved in safety, toxicity and the like.
The compound of the present invention is useful for the suppression of postprandial hyperglycemia in the prediabetic state, the treatment of non-insulin-dependent diabetes mellitus, the treatment of autoimmune diseases such as joint inflammation or rheumatoid arthritis, the treatment of intestinal mucosal diseases, the promotion of growth, the prevention of rejection of a transplanted organ, the treatment of obesity, the treatment of an eating disorder, the treatment of HIV infection, the suppression of cancer metastasis, the treatment of prostatic hypertrophy, the treatment of periodontitis and the treatment of osteoporosis.

## Claims

1. 6-[(3R)-3-aminopiperidin-1-yl]-5-(2-chloro-5-fluorobenzyl)-1,3-dimethyl-1H-pyrrolo[3,2-d]pyrimidine-2,4(3H,5H)-dione monohydrochloride hemihydrate.

2. The compound according to claim 1, showing main peaks at diffraction angles (20) in powder X-ray diffraction: 8.6±0.2°, 14.9±0.2° and 15.4±0.2°.

3. The compound according to claim 1, showing main peaks at diffraction angles (2θ) in powder X-ray diffraction: 4.3±0.2°, 8.6±0.2°, 12.9±0.2°, 14.9±0.2°, 15.4±0.2°, 17.1±0.2°, 18.7±0.2°, 23.0±0.2°, 25.2±0.2°, 26.4±0.2°, 30.3±0.2° and 34.7±0.2°.

4. A medicament containing a compound according to any one of claims 1 to 3 as an active ingredient.

5. A dipeptidyl peptidase-IV inhibitor containing a compound according to any one of claims 1 to 3 as an active ingredient.

6. A therapeutic agent for diabetes containing a compound according to any one of claims 1 to 3 as an active ingredient.

7. A method of treating diabetes comprising administering an effective amount of a compound according to any one of claims 1 to 3 to a patient requiring treatment.
